# EUROPEAN PATENT SPECIFICATION

(11) **EP 1 595 566 B1**
(45) Date of publication and mention of the grant of the patent: **02.07.2008**
(21) Application number: 05252788.4
(22) Date of filing: 06.05.2005
(51) Int. Cl.: A61M 25/00, A61F 2/00

(54) **Catheter packaging with integrated clip**
Katheterverpackung mit einem integriertem Klipp
Emballage pour cathéter avec un clip intégré

(30) Priority: 12.05.2004 US 570511 P; 30.09.2004 US 954712
(43) Date of publication of application: 16.11.2005
(73) Proprietor: Cordis Corporation, Miami Lakes, Florida 33014 (US)
(72) Inventor: Rispens, Johan Dirk, 9331 JS Norg (NL)
(74) Representative: Belcher, Simon James

(56) References cited:
- WO-A-98/18515
- WO-A-03/026535
- US-A- 5 344 011
- US-A1- 2002 130 059

## Description

The present invention relates to catheter packaging, and more particularly to catheter packaging with a tubular structure having an integrated clip for releasably holding a portion of a catheter.

Catheters generally have a flexible shaft extending between a proximal end and a distal end, such that the shaft can follow a curved or tortuous path. A hub is often affixed to the catheter shaft proximal end. The hub may serve a variety of functions, including providing a handle for manipulating the catheter, and/or defining a proximal port(s) communicating with one or more tubular passages or lumens that may be defined by the catheter shaft. The hub may also include one or more luer-lock fittings or other features for coupling the hub and lumen(s) to various equipment.

Of course, catheter packaging may be used with many different kinds and types of catheters, including for example balloon catheters, diagnostic catheters, guiding catheters, stent delivery system catheters, injection catheters, gene therapy catheters, electrophysiology catheters, therapeutic drug delivery catheters, ultrasound catheters, laser angioplasty catheters, etc.

Catheters are often provided in packaging to protect the catheter during sterilization, shipment, storage, etc. Such packaging may include a tubular structure into which the catheter is inserted. This tubular structure may have a shape that is straight, curved, helical, or a spiral coil. Also, the tubular structure may be held within or be affixed to a reinforcing structure. Different portions of the tubular structure may be held in relative positions by a fastener, or may be affixed together using an adhesive, heat seal, or welding. For example, the loops of a tubular spiral coil structure may be held together with one or more clips or by being welded, to hold the spiral loops in position.

After the catheter is inserted within the tubular structure, it is desirable to provide some feature to hold the catheter in position inside the tubular structure, and resist movement of the catheter to slide out of the tubular structure. Accordingly, a separate clip may be attached to the tubular structure, to hold the catheter in place within the tubular structure, and this clip may be designed to clip onto a portion of the hub.

WO-A-03/026535 discusses a carrier tube for an intravascular device. In one example, a removable clip engages an opening defined in a tubular packaging wall and mates with grooves in a hub assembly to releasably connect the tubular packaging on an intravascular device.

Such a retainer should be not only effective in holding the catheter in position within the tubular packaging, but also be easy to disengage and remove the catheter from the tubular packaging in preparation for use. It is also desirable to provide a retainer that is inexpensive and easy to manufacture.

According to the present invention, there is provided a catheter packaging system as defined in appended claim 1.

The present invention relates to packaging systems for any suitable type of catheter, in which the packaging includes a tubular structure with an integrated clip. In other words, catheter packaging according to the present invention includes packaging for various types of catheters, for example balloon catheters, diagnostic catheters, guiding catheters, stent delivery system catheters, injection catheters, gene therapy catheters, electrophysiology catheters, therapeutic drug delivery catheters, etc.

The terms "tube" and "tubular" are used in their broadest sense, to encompass any structure arranged a radial distance around a longitudinal axis. Accordingly, the terms "tube" and "tubular" include any structure that (i) is cylindrical or not, such as for example an elliptical or polygonal cross-section, or any other regular or irregular cross-section; (ii) has a different or changing cross-section along its length; (iii) is arranged around a straight, curving, bent or discontinuous longitudinal axis; (iv) has an imperforate surface, or a periodic or other perforate, irregular or gapped surface or cross-section; (v) is spaced uniformly or irregularly, including being spaced varying radial distances from the longitudinal axis; or (vi) has any desired combination of length or cross-sectional size.

The term "clip" is also used in its broadest sense, to encompass any releasable clip, fastener, hook, grasping or clasping member for holding a catheter in a tubular structure until released.

Catheter packaging systems along the lines of the present invention may provide several advantages. In an example, catheter packaging including a tubular structure and catheter clip may be made of a single piece, which provides advantages including simplicity. An integrated clip is simpler and easier to manufacture, and provides cost savings.

In addition, it is possible that the physician or health professional may be able to grasp the hub of the catheter, disengage it from the clip, and withdraw the catheter from the packaging, all in a smooth movement and primarily using one hand.

Any suitable material may be used to make catheter packaging, including polymers and other materials suitable for packaging medical devices.

Also, catheter packaging may be used with or enclosed in other packaging items. For example, the catheter and tubular structure may be placed in a polymer tray, and/or may be enclosed by an envelope of polymer sheets or metal foil. The envelope may likewise be enclosed within an outer box, made of cardboard or other suitable material. Whatever the outermost component of the catheter packaging system is, it will generally carry the labeling for the medical device.

It is of course possible to build various kinds and designs of catheter packaging according to the present invention, by various techniques and of various materials, to obtain the desired features. It should be noted that the present invention also relates to methods for making catheter packaging, and methods for using catheters and catheter packaging, during or in preparation for medical treatment of a patient.

The catheter packaging system can be prepared by a process comprising the steps of:
providing a catheter having a flexible shaft extending from a proximal end to a distal end, with a hub affixed to the proximal end; wherein the hub defines a body having a first size and a neck having a second smaller size;
providing a tubular component with a sidewall and which defines a lumen and a tubular size, such that the catheter shaft fits within the lumen of the tubular component; forming an aperture in the sidewall of the tubular component, such that the hub body may be partially received in the opening of the tubular component, wherein the first size of the hub body is greater than the size of the tubular component opening;
cutting or removing a portion of the collar, to form at least one clip; the clip having a relaxed position and being moveable to an opened position, tending to resiliently return to the relaxed position;
inserting the catheter shaft into the tubular component, positioning the catheter hub partially in the aperture and near the clip; and
opening the clip to its open position, and inserting the neck of the catheter hub into the clip, and allowing the clip to resiliently clasp the neck of the catheter hub, such that the hub body is partially received within and extends out of the aperture, such that the clip tends to hold the catheter in position within the packaging system.

Embodiments of the present invention will now be described by way of example only with reference to the accompanying drawings, in which:
Figure 1 is a partial plan view of a catheter and packaging;
Figure 2 is a partial side elevation view of the catheter and packaging of Figure 1;
Figure 3 is a perspective view of a catheter and packaging;
Figure 4 is a partial cross-section view of packaging;
Figures 5 is a partial plan view of packaging;
Figures 6 and 7 are partial perspective views of a catheter and packaging;
Figures 8 and 9 are partial plan and elevation views of a catheter and packaging;
Figure 10 is a partial perspective view of a catheter and packaging; and
Figures 11-13 are cross-section views of the catheter and packaging of Figure 8.

The following description of the preferred embodiments of the present invention is merely illustrative in nature, and as such it does not limit in any way the present invention, its application, or uses.

The drawings depict catheter packaging along the lines of the present invention. An example is catheter packaging 10, including a tube 12 with an opening 14 in the sidewall of tubular structure 12, and an integrated clip having a first and second clasping member 16 and 18.

A catheter 20 is shown in position inside the catheter packaging. For purposes of the present invention, many types of catheters may be used. Indeed, catheter packaging may be designed to accommodate any catheter having features that lend themselves to catheter packaging according to the present invention.

The catheter 20 in the drawings has a flexible shaft extending from a proximal end to a distal end, and a hub affixed to the proximal end of the catheter shaft. The shaft is sufficiently flexible and has a size selected to fit within a passage or lumen defined by tubular structure 12.

The hub has a body with a first size or dimension larger than or extending outward beyond the outer size of the catheter shaft and the tubular structure 12. The hub also has a neck with a second size that is smaller than the hub body, as well as a proximal end feature that is larger than the neck.

In examples shown in the drawings, the hub body has outwardly extending features such as wings 22 and 24, and a port or luer-lock 26, with a smaller neck portion between luer-lock 26 and the wings 22 and 24.

In the tubular structure of catheter packaging, the integrated clip may be formed by removing a portion of the sidewall of the tubular structure 12 to form opening 14, such that wings 22 and 24 extend out of the opening 14. Opening 14 may be formed as a scalloped portion removed from one side of the tubular component 12.

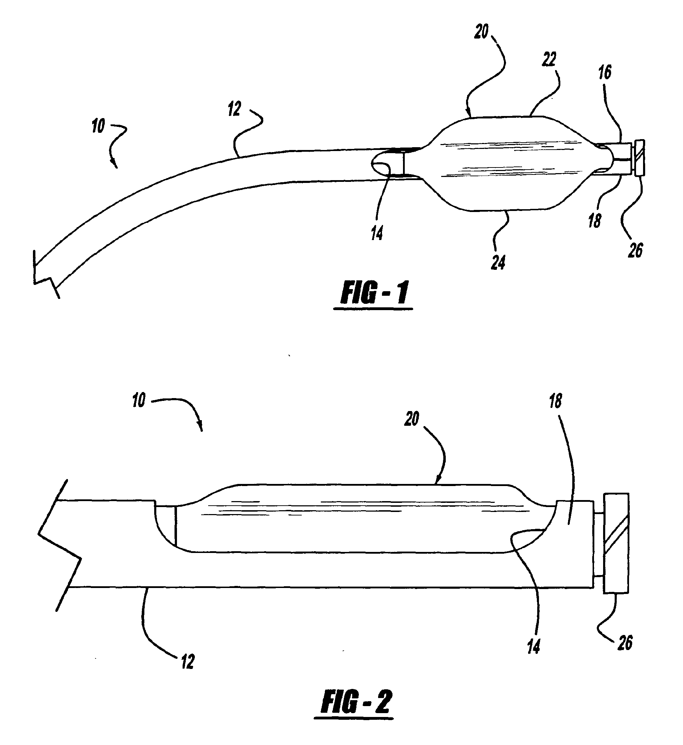

At least one clasping member is formed in the tubular structure for grasping a portion of the hub, in this case the neck portion. In Figures 1-5, first and second clasping members 16 and 18 are formed by cutting a longitudinal slit through a tubular collar at one end of tubular structure 12. The packaging thus has an integrated clip for holding the catheter hub in position.

The first and second clasping members 16 and 18 are defined by moveable portions of the tubular component, and they tend to resiliently move from an open position to a clasping position. Clasping members 16 and 18 may be releasably clasped around the neck of the catheter hub, such that the clip is integral and unitary with a remainder of tubular structure 12.

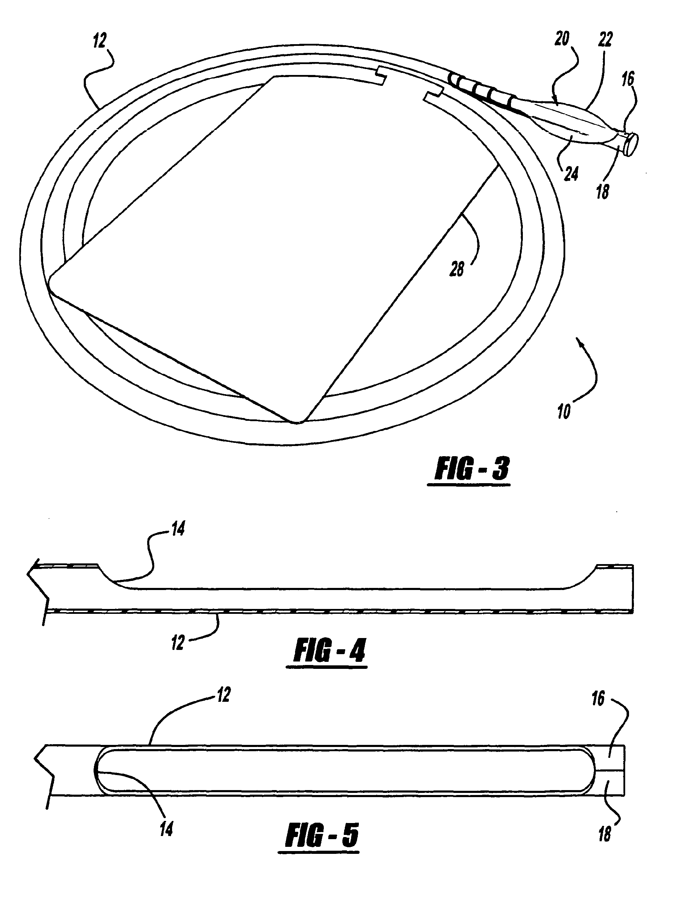

Additional packaging materials may of course be used, such as mounting card 28, shown in Figure 3.

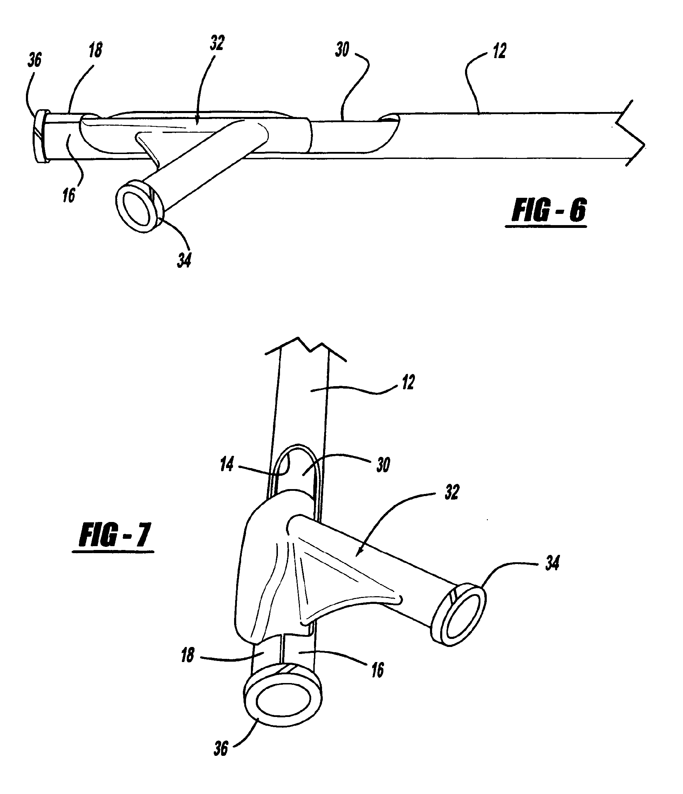

A second type of catheter is shown in Figures 6-7 and 10, which defines two passages or lumens, and has two corresponding proximal ports, such as for example an over-the-wire balloon catheter. This balloon catheter has a shaft 30, a proximal hub 32 which defines an inflation port 34 and a guidewire port 36. The catheter packaging shown in Figures 6 and 7 therefore accommodates such a balloon catheter having more than one proximal port.

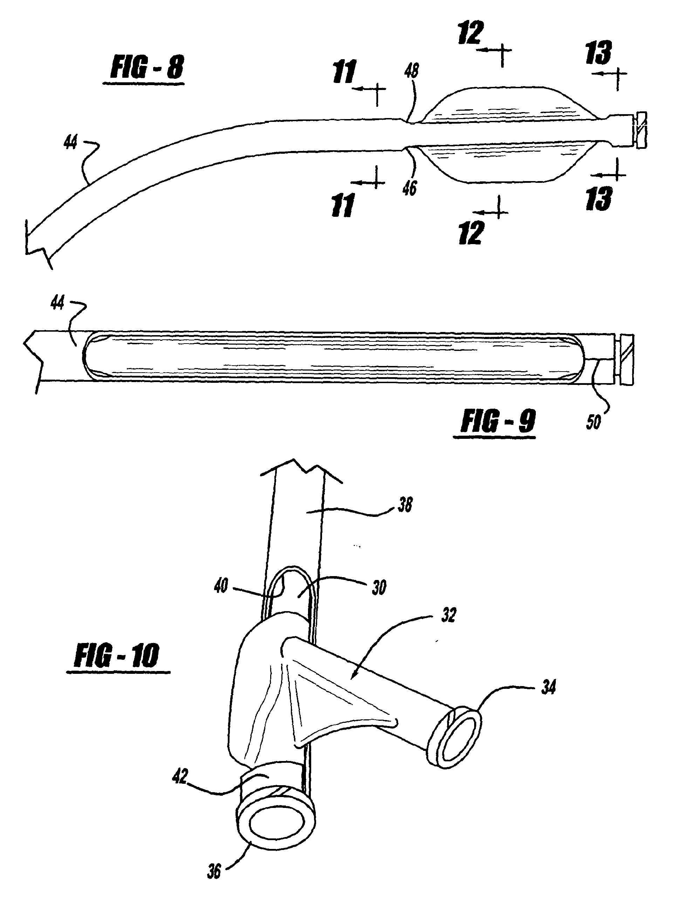

Of course, as shown in Figure 10, catheter packaging along the lines of the present invention includes a packaging tube 38 with an opening 40 and a single clasp 42, rather than multiple clasping members.

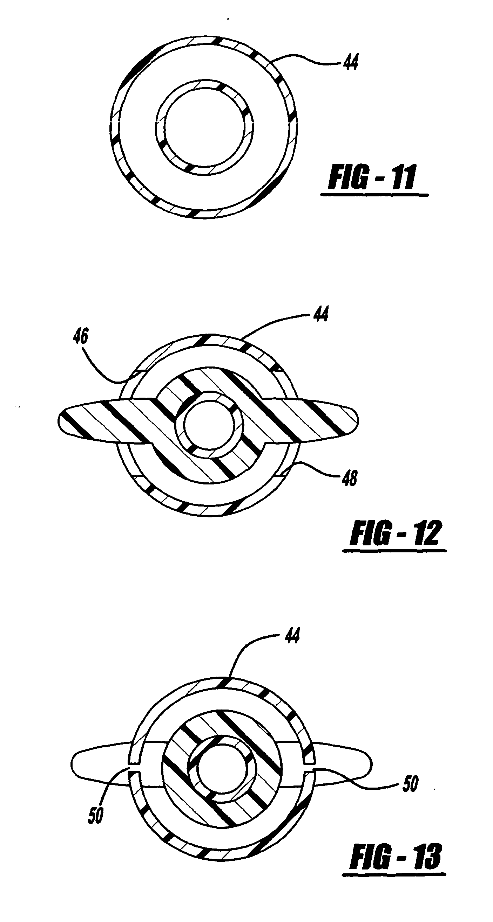

Another arrangement of catheter packaging may include multiple openings, as shown in Figures 8-9 and 11-13. Packaging tube 44 has a first and second opening 46 and 48, and longitudinal slits 50 in the tube 44, to allow the catheter to be removed from the packaging.

Many different materials may be used for manufacturing the catheter packaging of the present invention, including the tubular component(s) and integrated clip. For example, various polymers may be used.

Accordingly, catheter packaging according to the principles of the present invention may be made of any suitable material using a variety of methods. Various polymers have the desired characteristics of strength, resilience, flexibility, biocompatibility and endurance.

Catheter packaging along the lines of the present invention may provide several advantages. For example, a tubular structure and catheter clip may be made of a single unitary piece of material, which provides advantages including simplicity. An integrated clip is simpler and easier to manufacture, and provides cost savings. It is possible that the physician or health professional may be able to grasp the hub of the catheter, disengage it from the clip, and withdraw the catheter from the packaging, all in a smooth movement and primarily using one hand.

It should be understood that an unlimited number of configurations for the present invention could be realized. The foregoing discussion describes merely exemplary embodiments illustrating the principles of the present invention, the scope of which is recited in the following claims.

## Claims

1. A catheter packaging system, comprising:
a catheter (20) having a flexible shaft extending from a proximal end to a distal end, with a hub affixed to the proximal end, wherein the hub defines a body having a first size and a neck having a second smaller size;
and packaging (10) comprising:
a tubular component (12, 38, 44) with a sidewall and defining a lumen and a tubular size, such that the catheter shaft may be inserted within the lumen of the tubular component (12, 38, 44);
an opening (14, 40, 46, 48) formed in the sidewall of the tubular component (12, 38, 44), such that the hub body may be partially received in the opening of the tubular component (12, 38, 44), wherein the first size of the hub body is greater than the size of the tubular component opening (14, 40, 46, 48);
at least one clip defined by a moveable portion (16, 18, 42) of the tubular component, such that the at least one clip may be releasably clasped around the neck of the catheter hub, wherein the tubular component (12, 38, 44) and the at least one clip are integral and unitary;
such that the packaging tubular component (12, 38, 44) has an integrated clip for releasably holding a portion of the catheter hub.

2. The packaging system of Claim 1, wherein the packaging further comprises a second clip.

3. The packaging system of Claim 1, wherein the tubular component (12, 38, 44) and clip are made of polymer material.

4. The packaging system of Claim 1, wherein the opening (14, 40, 46, 48) is formed as a scalloped portion removed from one side of the tubular component (12, 38, 44).

5. The packaging system of Claim 1, wherein the at least one clip is formed by a collar having a slit (50).

6. The packaging system of Claim 1, wherein the catheter (20) is of a type selected from among the group of balloon catheters, diagnostic catheters, guiding catheters, stent delivery system catheters, injection catheters, gene therapy catheters, electrophysiology catheters, therapeutic drug delivery catheters, ultrasound catheters, and laser angioplasty catheters.

7. The packaging system of Claim 1, wherein the tubular component (12, 38, 44) has a shape selected from among the group of straight, curved, helical, and a spiral coil.

8. The packaging system of Claim 1, wherein the tubular component (12, 38, 44) further comprises a second aperture (48), and the hub has a pair of radially extending wings, such that the apertures (46, 48) are adapted to receive the wings.

9. The packaging system of Claim 1, wherein the clip is a clasping member.

## Patentansprüche

1. Katheterverpackungssystem, das Folgendes umfasst:
einen Katheter (20) mit einem flexiblen Schaft, der sich von einem proximalen Ende zu einem distalen Ende erstreckt, wobei ein Hub an dem proximalen Ende befestigt ist und wobei der Hub einen Körper mit einer ersten Größe und einen Hals mit einer zweiten kleineren Größe definiert;
und eine Verpackung (10), die Folgendes umfasst:
eine Röhrenkomponente (12, 38, 44) mit einer Seitenwand, die ein Lumen und eine Röhrengröße definiert, so dass der Katheterschaft in das Lumen der Röhrenkomponente (12, 38, 44) eingeführt werden kann;
eine Öffnung (14, 40, 46, 48), die in der Seitenwand der Röhrenkomponente (12, 38, 44) ausgebildet ist, so dass der Hubkörper teilweise in der Öffnung der Röhrenkomponente aufgenommen werden kann, wobei die erste Größe des Hubkörpers größer ist als die Größe der Röhrenkomponentenöffnung (14, 40, 46, 48);
wenigstens einen Clip, welcher durch einen beweglichen Abschnitt (16, 18, 42) der Röhrenkomponente definiert ist, so dass der wenigstens eine Clip lösbar den Hals des Katheterhubs umklammern kann, wobei die Röhrenkomponente (12, 38, 44) und der wenigstens eine Clip integral und einheitlich sind;
derart, dass die Verpackungsröhrenkomponente (12, 38, 44) einen integrierten Clip zum lösbaren Halten eines Abschnittes des Katheterhubs besitzt.

2. Verpackungssystem nach Anspruch 1, bei welchem die Verpackung weiterhin einen zweiten Clip umfasst.

3. Verpackungssystem nach Anspruch 1, bei welchem die Röhrenkomponente (12, 38, 44) und der Clip aus einem Polymermaterial hergestellt sind.

4. Verpackungssystem nach Anspruch 1, bei welchem die Öffnung (14, 40, 46, 48) als ein muschelförmiger Abschnitt gebildet ist, der von einer Seite der Röhrenkomponente (12, 38, 44) entfernt ist.

5. Verpackungssystem nach Anspruch 1, bei welchem der wenigstens eine Clip durch eine Manschette mit einem Schlitz (50) gebildet ist.

6. Verpackungssystem nach Anspruch 1, bei welchem der Katheter (20) eine Ausführung ist, die aus einer Gruppe ausgewählt ist von Ballonkathetern, diagnostischen Kathetern, Führungskathetern, Stentszuführungssystemkathetern, Injektionskathetern, Gentherapiekathetem, elektrophysikalische Kathetern, Zuführungskathetern für therapeutische Medikamente, Ultraschallkathetern und Laserangioplastiekatheten.

7. Verpackungssystem nach Anspruch 1, bei welchem die Röhrenkomponente (12, 38, 44) eine Form aufweist aus einer Gruppe von gerade, gekrümmt, helikal und einer Spiralspule.

8. Verpackungssystem nach Anspruch 1, bei welchem die Röhrenkomponente (12, 38, 44) weiterhin eine zweite Öffnung (48) umfasst und der Hub ein Paar von sich radial erstreckenden Flügeln aufweist, derart, dass die Öffnungen (46, 48) zum Aufnehmen der Flügel eingerichtet sind.

9. Verpackungssystem nach Anspruch 1, bei welchem der Clip ein Klammerelement ist.

## Revendications

1. Système d'emballage de cathéter, comprenant :
un cathéter (20) ayant un arbre flexible s'étendant d'une extrémité proximale à une extrémité distale, avec un moyeu fixé à l'extrémité proximale, dans lequel le moyeu définit un corps ayant une première taille et un col ayant une seconde taille plus petite :
et l'emballage (10) comprenant :
un composant tubulaire (12, 38, 44) avec une paroi latérale et définissant une lumière et une taille tubulaire, de sorte que l'arbre de cathéter puisse être inséré dans la lumière du composant tubulaire (12, 38, 44) ;
une ouverture (14, 40, 46, 48) ménagée dans la paroi latérale du composant tubulaire (12, 38, 44), de sorte que le corps de moyeu puisse être partiellement reçue dans l'ouverture du composant tubulaire (12, 38, 44), dans lequel la première taille du corps de moyeu est supérieure à la taille de l'ouverture de composant tubulaire (14, 40, 46, 48) ;
au moins un clip défini par une portion mobile (16, 18, 42) du composant tubulaire, de sorte que l'au moins un clip puisse être agrafé de manière amovible autour du col du moyeu de cathéter, dans lequel le composant tubulaire (12, 38, 44) et l'au moins un clip sont réalisés en un seul morceau unitaire ;
de sorte que le composant tubulaire d'emballage (12, 38, 44) a un clip intégré pour retenir de manière amovible une portion du moyeu de cathéter.

2. Système d'emballage selon la revendication 1, dans lequel l'emballage comprend en outre un second clip.

3. Système d'emballage selon la revendication 1, dans lequel le composant tubulaire (12, 38, 44) et un clip sont faits de matériau polymère.

4. Système d'emballage selon la revendication 1, dans lequel l'ouverture (14, 40, 46, 48) est formée comme une portion crantée, enlevée d'un côté du composant tubulaire (12, 38, 44).

5. Système d'emballage selon la revendication 1, dans lequel l'au moins un clip est formé par un collier doté d'une fente (50).

6. Système d'emballage selon la revendication 1, dans lequel le cathéter (20) est d'un type choisi parmi le groupe de cathéters à ballon, de cathéters de diagnostic, de cathéters de guidage, de cathéters de système de distribution de stent, de cathéters d'injection, de cathéters de traitement génique, de cathéters d'électrophysiologie, de cathéters de distribution de médicament thérapeutique, de cathéters à ultrasons, et de cathéters d'angioplastie laser.

7. Système d'emballage selon la revendication 1, dans lequel le composant tubulaire (12, 38, 44) a une forme droite, courbe, hélicoïdale ou de bobine à spirale.

8. Système d'emballage selon la revendication 1, dans lequel le composant tubulaire (12, 38, 44) comprend en outre une seconde ouverture (48) et le moyeu dispose d'une paire d'ailettes s'étendant radialement de sorte que les ouvertures (46, 48) sont adaptées pour recevoir les ailettes.

9. Système d'emballage selon la revendication 1, dans lequel le clip est un élément d'agrafage.
